# EUROPEAN PATENT APPLICATION

(11) **EP 4 226 950 A1**
(43) Date of publication of application: **16.08.2023**
(21) Application number: 23156468.3
(22) Date of filing: 14.02.2023
(51) Int. Cl.: A61L 2/00, A61L 2/10, A61L 2/18, A61L 2/22, A61L 2/24

(54) **A SYSTEM FOR STERILIZING AND DRYING A USER'S HANDS**

(30) Priority: 15.02.2022 DK PA202200124
(71) Applicant: Grand Device, 3550 Slangerup (DK)
(72) Inventor: Løfgren-Granding, Bertram, 3550 Slangerup (DK)
(74) Representative: Larsen & Birkeholm A/S

(57) **Abstract**

The invention relates to a system for sterilizing and drying a user's hands, said system comprises: a housing having a sterilizing chamber provided with at least one opening for insertion of one or two hands, said housing comprising a sterilization system comprising: an antiseptic solution spraying system comprising a high-pressure pump for pumping the antiseptic solution from an antiseptic storage compartment to a nozzle system inside said sterilizing chamber. The sterilization system further comprises a UV-unit having at least one UV light emitter for the emitting of a hand situated inside said sterilizing chamber and a detection system capable of detecting the presence of a hand inside said sterilizing chamber. The system further comprises a programable controller being connected to the detection system and to said sterilizing system and a timer connected to the programmable controller. The programmable controller being programmed such that it starts the timer and a sterilizing- and drying process when the presence of a hand is detected by said detection system.

## Description

### Field of the Invention

The invention relates to a system for sterilizing and drying a user's hands, said system comprises: a housing having a sterilizing chamber provided with at least one opening for insertion of one or two hands, said housing comprising a sterilization system comprising: an antiseptic solution spraying system comprising a high-pressure pump for pumping the antiseptic solution from an antiseptic storage compartment to a nozzle system inside said sterilizing chamber, said sterilization system further comprises a UV-unit having at least one UV light emitter for the emitting of a hand situated inside said sterilizing chamber; a detection system capable of detecting the presence of a hand inside said sterilizing chamber; a programable controller being connected to the detection system and to said sterilizing system, a timer connected to the programable controller.

### Background of the Invention

Infectious diseases are caused by various pathogens: virus, bacteria, fungus, spores, etc. Once on or within the body they replicate and ultimately can cause infection and illness, sometimes resulting in death. Pathogens reach the body through contaminated food, aerosolized pathogens in air or on dust, human contact with surfaces or human-to-human contact. Hands are a significant distributor of diseases. Thus, hand washing is one important means of preventing the spread of infection and germs that can cause colds and flus.

In a hospital or other health care environments, health care workers and physicians are significant factors in disease transmission from patient to patient by virtue of inadequate attention to, or inadequate technology for, hand sanitation.

Extended application time improves the protection. For example, surgeons scrub for many minutes to improve the removal of pathogens, and nurses and other healthcare workers wash their hands frequently, and as a result, cause their hands to become painfully sore thereby making it difficult to practice this technique consistently. Thus, as the required application time increases and the unpleasant side effects increase, compliance with sanitization procedures decreases. Surgeons may not scrub for the necessary length of time-approximately 10 minutes-or with the necessary vigor, and nurses may avoid hand washing to reduce the discomfort associated with the hand irritation that can be exacerbated from wearing problems. As health care professionals go from patient to patient, they can transport pathogens on the surface of the gloves just as they can on bare hands. Furthermore, one touch of any surface by the hand contaminates the hand. All the effort at sanitation can be lost by a single touch by the hand to a surface or by settling of aerosols containing pathogens or dry pathogens drifting in the air. The contaminated hand is a major distributor of transmission of pathogens between humans, and is possibly the primary source of hospital acquired infection spread. Furthermore, the gloves cannot be easily washed while being worm, the gloves are not replaced as often as should be to limit the transmission of disease and constant replacement of gloves increases costs associated with patient care. It is generally understood that the purpose of the gloves is to protect the healthcare worker from the patient, not the patient from the healthcare worker.

The necessary time for effective use of alcohol including drying is tens of seconds. One factor that is cited as a significant inconvenience is the time required to achieve substantial reduction of the number of pathogens (e.g., 99.99%), or missed areas and thus the process of sanitation is frequently bypassed. Alcohol rubs are ineffective on spores. Another factor is that the use of alcohol dries the epidermis, which is supposed to function to protect the moisture of the skin. Hence the skin sometimes can become irritated and the procedure is bypassed.

Bare hands are also a major element in the spread of infection in schools. The cost to schools of absence is very high. Students can miss class time and carry illnesses home. Hence, proper hand sanitation in the school environment is also financially important to the schools, to the students and to the parents. Washing hands is typically not practiced as frequently as desired or in a sufficient manner. Moreover, in many developing countries, the sanitary and hygienic conditions at schools are often very poorand can be characterized by the absence of properly functioning or existing water supply for sanitation or hand washing facilities. Studies have demonstrated that the absentee rate is reduced by 50% with proper hand washing.

Clean hands in restaurant settings are similarly critical to prevent the spread of disease. FDA reports that poor personal hygiene in a food service environment is a critical area that needs immediate attention.

Ultra-violet (UV) irradiation can be used to deactivate many pathogens. However, time and intensity are crucial to the effectiveness of UV irradiation.

From US5074322 A is known, a sterilizing hand dryer comprising an antiseptic solution spraying system for discharging antiseptic solution through a nozzle to sterilize the body limb inserted in a sterilizing chamber and detected by an electric eye, a hot air blower means for discharging hot air in said sterilizing chamber for drying said body limb, a control circuit for stopping the sterilizing hand dryer 3 seconds after removal of said body limb from said sterilizing air bath camber.

### Object of the Invention

It is an object of the invention to provide an improved sterilizing system.

### Summary of the Invention

This is achieved by said programable controller being programmed such that it starts the timer and a sterilizing- and drying process, when the presence of a hand is detected by said detection system, said sterilizing and drying process comprise the steps of: activating the antiseptic solution spraying system after a first period of time (T1) after the detection in order to spray antiseptic solution on the hand; stopping the antiseptic solution spraying system after a second period of time (T2) after the detection; activating the UV-unit after a third period of time (T3) after the detection in order to dry the hand by aid of UV-light; stopping the UV-unit after a fourth period of time (T4) after the detection.

By giving the system an opportunity to combine the effect of alcohol with the effect of UV light, an improved sterilization is obtained compared to situations where these sterilants are used alone.

By designing the chamber with nozzles, it is further achieved that the sterilizing liquid is atomized into the chamber, a high guarantee is obtained that all areas on the hands inserted in the apparatus come into contact with the atomization during the entire time that the atomization is in the chamber. In addition to this, the UV light achieves a further evaporation of the alcohol that is still in the air as liquid-drops and even further a very fast drying of the hands.

Other embodiments are recited in the dependent claims.

### BRIEF DESCRIPTION OF THE DRAWINGS

In the following an embodiment of the invention will be described with reference to the figures, wherein:
fig. 1 shows - a cross section of the system for sterilizing and drying hands, the cross section is shown from the side.
Fig. 2 shows - a cross section of the system for sterilizing and drying hands, the cross section is shown from the top.

### Detailed description of the invention

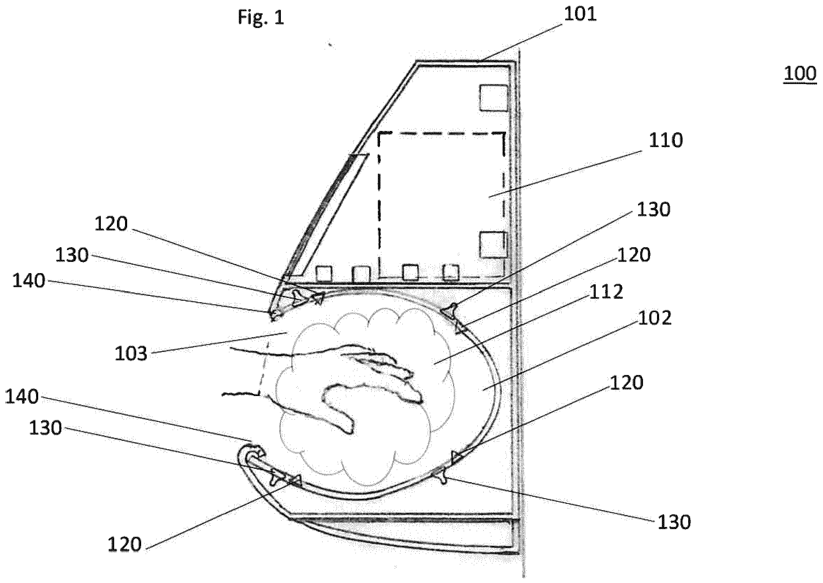

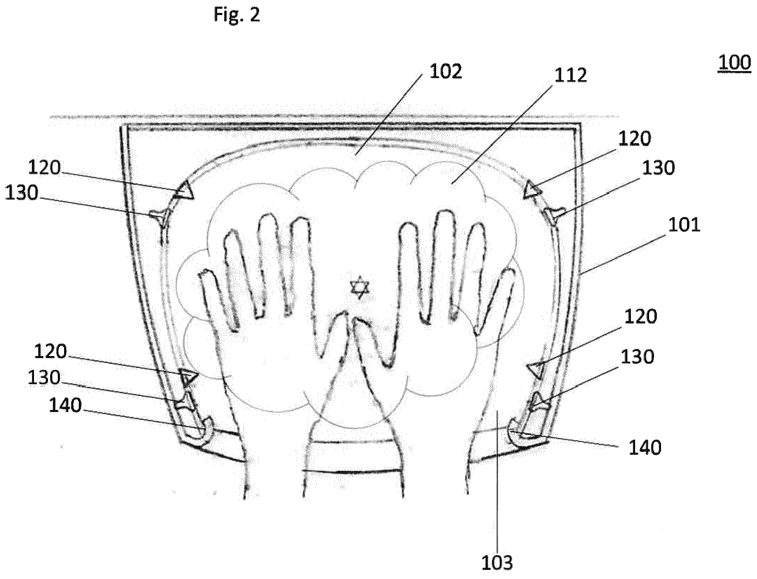

Referring to figs. 1-2 showing a cross section of the system for sterilizing and drying hands 100. The system for sterilizing and drying 100 comprises a housing 101, and inside the housing 100 a sterilizing chamber 102 is situated. The sterilizing chamber is provided with at least one opening 103 for insertion of one or two hands. Inside the housing a sterilization system is provided.

The sterilizing system comprises an antiseptic solution spraying system 110 having a high-pressure pump for pumping the antiseptic solution from an antiseptic storage compartment to a nozzle system 120 inside said sterilizing chamber. The sterilization system further comprises a UV-unit having at least one UV light emitter 130 for the emitting of a hand situated inside said sterilizing chamber. Further the system comprises a detection system 140 capable of detecting the presence of a hand inside said sterilizing chamber. Typically, such a detector will be an infrared sensor, but can of course also be made in many other ways. For example, as a sensor that reacts when a light beam is interrupted, or as a touch switch.

A programable controller is connected to the detection system and to said sterilizing system and a timer is connected to the programmable controller. The programmable controller is programmed such that it starts the timer and a sterilizing- and drying process when the presence of a hand is detected by said detection system.

The sterilizing- and drying process comprise the steps of: activating the antiseptic solution spraying system after a first period of time (T1) after the detection in order to spray antiseptic solution on the hand; stopping the antiseptic solution spraying system after a second period of time (T2) after the detection; activating the UV-unit after a third period of time (T3) after the detection in order to dry the hand by aid of UV-light, stopping the UV-unit after a fourth period of time (T4) after the detection.

Normally, the processor will start the antiseptic solution spraying system immediately after a hand is detected. However, in one embodiment, the spraying process can be omitted altogether and one way to do this in practice is by setting the time unit for T1, i.e. the time that must elapse before the antiseptic solution spraying system is activated to be longer than the time T4 which is the time that must elapse before the UV light unit stops. In order to avoid the system in these situations starting a spray cycle, it will usually be made such that the system resets and stops the timer when the time T4 has elapsed.

Another way to avoid a spray cycle is by setting the first time period T1 to be substantially the same as the time period T2.

When the spray system thus is put out of action, the system will act as a hand dryer that dries by means of UV light. In all embodiments of the invention, it will be preferred that the UV light is UV-B light.

### REFERENCES

- 100: System for sterilizing and drying
- 101: Housing
- 102: Sterilizing chamber
- 103: Opening
- 110: Antiseptic solution spraying system
- 111: High-pressure pump
- 112: Antiseptic solution spray
- 113: Storage compartment
- 120: Nozzle system
- 130: UV-unit
- 140: Detection system

## Claims

1. A system for sterilizing and drying a user's hands (100), said system comprises:
• a housing (101) having a sterilizing chamber (102) provided with at least one opening (103) for insertion of one or two hands, said housing comprising a sterilization system comprising: an antiseptic solution spraying system (110) comprising a high-pressure pump for pumping the antiseptic solution from an antiseptic storage compartment to a nozzle system (120) inside said sterilizing camber, said sterilization system further comprises a UV-unit (130) having at least one UV light emitter for the emitting of a hand situated inside said sterilizing chamber;
• a detection system (140) capable of detecting the presence of a hand inside said sterilizing chamber;
• a programable controller being connected to the detection system and to said sterilizing system;
• a timer connected to the programmable controller;
**characterized in that**, said programmable controller being programmed such that it starts the timer and a sterilizing- and drying process, when the presence of a hand is detected by said detection system, said sterilizing- and drying process comprise the steps of:
• activating the antiseptic solution spraying system after a first period of time (T1) after the detection in order to spray antiseptic solution on the hand;
• stopping the antiseptic solution spraying system after a second period of time (T2) after the detection;
• activating the UV-unit after a third period of time (T3) after the detection in order to dry the hand by aid of UV-light;
• stopping the UV-unit after a fourth period of time (T4) after the detection.

2. A system for sterilizing and drying a user's hands according to claim 1 wherein, said UV-unit comprises at least one UV-C and/or UV- B and/or UV-A light emitter.

3. A system for sterilizing and drying a user's hands according to claim 1 wherein, said system comprises an alarm being connected to said programmable controller such that it emits an alarm if the detection unit does not detect the presence of a hand prior to the end of the fourth period of time.

4. A system for sterilizing and drying a user's hands according to any of the claims 1 - 3 wherein, the fourth time period is longer than the third time period and the third time period is longer than the second time period and the second time period is longer than the first time period.

5. A system for sterilizing and drying a user's hands according to any of the claims 1-4 wherein,
• T4-T3 is between 0 and 85 sec.; preferably between 0-60 sec. and more preferably between 0-20 sec.
• T2-T1 is between 0-45 sec., preferably between 0-20sec.

6. A system for sterilizing and drying a user's hands according to any one of claims 1-5, wherein the antiseptic solution is a mixture of water and ethanol coming from a container within said antiseptic storage compartment.

7. A system for sterilizing and drying a user's hands according to any one of claims 1-5, wherein the antiseptic solution is a mixture of a first and a second liquid coming from a first and a second container inside the antiseptic storage space, wherein the first container essentially comprises the first liquid and the second container essentially comprises the second liquid, the mixing ratio between the first liquid and the second liquid is regulated by the programmable controller and the actual mixing of these liquids takes place before the antiseptic solution reaches the nozzle system.

8. A system for sterilizing and drying a user's hands according to any one of claims 1-5, wherein the antiseptic solution is a mixture of a first and a second liquid coming from a first and a second container inside the antiseptic storage space, wherein the first container essentially comprises the first liquid and the second container essentially comprises the second liquid, the mixing ratio between the first liquid and the second liquid is regulated by the programmable controller and the actual mixing of these liquids takes place in the sterilizing chamber after these liquids are sprayed into the sterilizing chamber by separate nozzles in the nozzle system, the separate nozzles spraying the first liquid or the second liquid respectively.

9. A system for sterilizing and drying a user's hands according to claim 6 or 7, wherein the first liquid is sterile water.

10. A system for sterilizing and drying a user's hands according to any of the claims 6-8, wherein the first liquid is sterile water, and the second liquid is essentially pure ethanol.

11. A system for sterilizing and drying a user's hands according to any of the claims 1-9, wherein the UV-unit comprises a light unit capable of emitting infrared light.
